# EUROPÄISCHE PATENTSCHRIFT

(11) **EP 1 267 937 B1**
(45) Veröffentlichungstag und Bekanntmachung des Hinweises auf die Patenterteilung: **22.04.2009**
(21) Anmeldenummer: 01914811.3
(22) Anmeldetag: 21.03.2001
(51) Int. Cl.: A61K 47/48

(54) **POLYSACCHARID-POLYPEPTID KONJUGAT**
POLYSACCHARIDE-POLYPEPTIDE CONJUGATE
CONJUGUE DE POLYSACCHARIDE ET DE POLYPEPTIDE

(30) Priorität: 21.03.2000 AT 4712000
(43) Veröffentlichungstag der Anmeldung: 02.01.2003
(73) Patentinhaber: Loibner, Hans, Dr., 1230 Wien (AT)
(72) Erfinder: LOIBNER, Hans, A-1238 Wien (AT); ECKERT, Helmut, CH-4104 Oberwil (CH)
(74) Vertreter: Pawloy, Peter Michael
(86) Internationale Anmeldenummer: PCT/AT2001/000079
(87) Internationale Veröffentlichungsnummer: WO 2001/070272

(56) Entgegenhaltungen:
- WO-A-96/20012
- WO-A-99/49897
- US-A- 6 011 008
- MEHVAR R: "Dextrans for targeted and sustained delivery of therapeutic and imaging agents" JOURNAL OF CONTROLLED RELEASE,ELSEVIER SCIENCE PUBLISHERS B.V. AMSTERDAM,NL, Bd. 69, Nr. 1, 3. Oktober 2000 (2000-10-03), Seiten 1-25, XP004217531 ISSN: 0168-3659
- DATABASE WPI Section Ch, Week 198050 Derwent Publications Ltd., London, GB; Class A96, AN 1980-89664C XP002168294 & SU 730 694 A (MEDIC ENZYMES ANTIB), 30. April 1980 (1980-04-30)

## Beschreibung

Die vorliegende Erfindung betrifft eine neuartige Verwendung von aufoxidierten Polysacchariden als Trägermaterial für Komponenten von Impfstoffen, insbesondere ein Verfahren zur Herstellung eines Polysaccharid-Polypeptid Konjugates durch Umsetzung eines Polysaccharids mit einem mindestens eine freie Aminogruppe enthaltenden Polypeptid sowie die Verwendung eines solchen Konjugates als Impfstoff.

Impfstoffe sind dadurch gekennzeichnet, dass ein oder mehrere Antigene in einer immunogenen Formulierung in kleiner Menge, meist parenteral (subkutan oder intramuskulär) verabreicht werden, um eine starke und protektive Immunantwort auszulösen. Die meisten Impfstoffe werden derzeit zum Schutz gegen mikrobielle Infektionen hergestellt. Die verwendeten Antigene sind in diesen Fällen inaktivierte und veränderte Mikroorganismen, oder Teile von solchen, oder definierte Proteine aus solchen Mikroorganismen, die geeignet sind, eine Immunantwort gegen den jeweiligen Mikroorganismus auszulösen.

Seit Jahren wird auch die Wirksamkeit vieler experimenteller Impfstoffe gegen andere Erkrankungen untersucht. Dazu gehören auch Impfstoffe gegen Krebs. Dabei soll das Immunsystem von Krebspatienten selektiv aktiviert werden, maligne Zellen zu bekämpfen. Das wird mittels verschiedenster Ansätze versucht. Dazu gehören Impfungen mit autologen oder allogenen Tumorzellen, chemisch oder molekularbiologisch modifizierten autologen oder allogenen Tumorzellen, isolierten oder mit Hilfe von chemischen oder molekularbiologischen Methoden hergestellten Tumor assoziierten Antigenen (TAA), daraus abgeleiteten Peptiden, anti-idiotypische Antikörper als Surrogat eines TAA, neuerdings auch Impfungen mit DNA, die für TAA oder daraus abgeleiteten Strukturen codieren, etc. Im Prinzip genügen sehr kleine Mengen eines geeigneten Impfstoffes, um für Monate bis Jahre eine Immunität zu induzieren, die bei Abschwächung durch Booster-Impfungen wieder aufgefrischt werden kann. Darüber hinaus kann bei aktiver Immunisierung sowohl eine humorale als auch eine zelluläre Immunität induziert werden, deren Zusammenspiel eine effektive Schutzwirkung gegen Krebs ergeben kann.

Zur Erreichung einer starken Immunität werden Antigene in Impfstoffen meistens zusammen mit einem Adjuvans verabreicht. Als Beispiele für Adjuvantien, jedoch nicht auf diese eingeschränkt, seien erwähnt: Aluminiumhydroxid (Alu-Gel), Derivate von Lipopolysaccharid, Bacillus Calmette Guerin (BCG), Liposomenbereitungen, Formulierungen mit zusätzlichen Antigenen, gegen die das Immunsystem bereits eine starke Immunantwort gemacht hat, wie z.B. Tetanus Toxoid, Pseudomonas Exotoxin, oder Bestandteile von Influenza-Viren, gegebenenfalls in einer Liposomenbereitung. Weiters ist bekannt, dass die Immunantwort auch durch die gleichzeitige Verabreichung von körpereigenen Proteinen verstärkt'werden kann, die eine wichtige Rolle beim Aufbau einer Immunantwort spielen, wie zum Beispiel Granutozyten-Makrophagen stimulierender Faktor (GM-CSF), Interleukin 2 (IL-2), Interleukin 12 (IL-12) oder Gamma Interferon (IFNγ).

Die US-5 554 730-B betrifft Polysaccharid-Protein Konjugate, wobei ein partikulärer Impfstoff geschaffen werden soll. Dafür wird primär durch Umsetzung eines Proteinträgers mit einem aufoxidierten Polysaccharid-Antigen in Gegenwart eines "crowding agents" (Wasserverdrängers) ein Polysaccharid-Protein Konjugat als Schiff'sche Base (Azomethin) geschaffen, wobei der Proteinträger aufgrund der Gegenwart des "crowding agents" sofort denaturiert und das Konjugat in Form von Mikropartikeln ausfällt. Ein Auflösen der ausgefällten Mikropartikeln in stark basischem Milieu (0,1 n NaOH) zum Erhalt einer Impflösung ist zwar prinzipiell möglich und auch geoffenbart, jedoch nur für den Fall der Verwendung eines Polysaccharid-Antigens sinnvoll, da aufgrund der Denaturierung ein allfälliges antigenes Protein seine antigenen Determinanten verloren hätte und daher nicht mehr wirksam wäre.

Die WO 99/55715 beschreibt Polysaccharid-Antigen Konjugate, wobei das Antigen entweder über einen geeigneten bivalenten Linker oder über eine endständige Aldehydgruppe an das Polysaccharid gebunden sind. Eine direkte Bindung des Antigens an das Polysaccharid über eine Azomethin-Bindung ist somit auf die Anzahl der im Polysaccharid vorhandenen endständigen Aldehydgruppen beschränkt.

Auch die DE-198 21 859-A1 beschreibt Polysaccharid-Antigen Konjugate, wobei ein geeigneter Crosslinker mittels Azomethin-Bindung an durch Perjodatoxidation erhaltene Aldehydfunktionen im Polysaccharid gebunden wird. Im Crosslinker wird zusätzlich eine Maleimidofunktion vorgesehen, an welche sich eine -SH Gruppe von Cystein addieren kann. Die zum Einsatz kommenden Antigene werden sodann N- oder C-terminal mit einem zusätzlichen Cys versehen, um die Addition der endständigen SH-Funktion mit dem Crosslinker und somit den Erhalt der beschriebenen Polysaccharid-Antigen Konjugate zu ermöglichen.

Die US-5 846 951 schließlich betrifft Polysaccharide mit mindestens 5 Sialinsäureresten, welche Polysaccharide mittels Oxidation mit Natriumperjodat an den nichtreduzierenden Enden der Polysialinsäuren mit endständigen Aldehydgruppen versehen werden können. Derart geschaffene endständige Aldehydgruppen können dann über Azomethin-Bindungen aminogruppenhältige Arzneimittel, beispielsweise Proteine, binden.

Die meisten Antigene, die für Impfstoffe Verwendung finden, beinhalten Strukturen mit primären Aminogruppen. Insbesonders haben alle Proteinantigene im Normalfall zumindestens ein, aber meistens mehrere Lysine in ihrer Aminosäuresequenz. Die Aminogruppen dieser Lysine liegen frei vor.

Es ist seit langem bekannt, dass primäre Amine eine Reaktion mit Aldehyden eingehen können. Das Produkt dieser Reaktion wird Schiff'sche Base genannt. Schiff'sche Basen sind nicht völlig stabile Verbindungen, sie können unter geeigneten Bedingungen hydrolisiert und damit in ihre Ausgangstoffe zurückgeführt werden.

Es ist weiters bekannt, dass Verbindungen, die vicinale Hydroxylgruppen beeinhalten, mit Hilfe von geeigneten Oxidantien, insbesondere mit Perjodsäure oder Salzen der Perjodsäure wie Natrium-meta-perjodat, so oxidiert werden können, dass unter Bruch der C-C Bindung, an denen die benachbarten Hydroxylgruppen sitzen, zwei Aldehydfunktionen entstehen.

Eine große Zahl von hochmolekularen Polysacchariden bestehen aus monomeren Zuckereinheiten, die vicinale Hydroxylgruppen tragen. Als zwei Beispiele, jedoch nicht auf diese eingeschränkt, seien Dextran und Mannan angeführt. Solche Polysaccharide können daher mit Perjodat in oben beschriebener Weise aufoxidiert werden, ohne dass die Bindungen zwischen den Monomeren gespalten werden. Wenn, bezogen auf die Zahl der Monomereinheiten, ein stöchiometrischer Unterschuss von Perjodat eingesetzt wird, findet die Oxidation nur partiell statt, das heißt, es werden nach einem Zufallsprinzip nur soviele Monomere aufoxidiert, wie der Menge von Perjodat entspricht.

Gemäß US 6 011 008 wird ein Verfahren zur Herstellung eines wasserlöslichen Polysaccharidkonjugats geoffenbart, wobei zwischen Aktivieren des Polysaccharids zu einem Dialdehyd durch Periodatoxidation und Bildung der Schiffschen Base (des Konjugats) zwingend ein Reinigungsschritt zur Befreiung des Dialdehyds von störenden Anionen und Nebenprodukten vorgesehen ist. Gemäß Spalte 1, Zeilen 49-60 liegt die Erfindung gemäß US 6 011 008 gerade eben in diesem Reinigungsschritt.

Der vorliegenden Erfindung liegt nun die Aufgabe zugrunde, weitere Mittel und Methoden zur Verfügung zu stellen, die zu immunogenen Formulierungen von Impfstoffen führen.

Diese Aufgabe wird bei einem Verfahren der eingangs angegebenen Art dadurch gelöst, dass ein vicinale Hydroxylgruppen aufweisender Polysaccharid-Träger unter Ringöffnung zur Schaffung von vicinalen Aldehydgruppen teilweise, vorzugsweise zumindest zu 20 %, bei stöchiometrischem Unterschuss eines Oxidationsmittels aufoxidiert und mit einem oder mehreren basenlabilen antigenen Polypeptid(en) enthaltend mindestens eine freie Aminogruppe umgesetzt wird, wobei das bzw. die Polypeptide über mindestens eine Azomethin-Bindung direkt an den Polysaccharid-Träger gebunden werden. Partiell aufoxidierte Polysaccharide stellen somit ein geeignetes Trägermaterial zur Formulierung von Impfstoffen dar, wenn die zum Einsatz kommenden basenlabilen antigenen Polypeptide ein oder mehrere freie primäre Aminogruppen enthalten und so mit den im Trägermaterial durch Ringöffnung geschaffenen vicinalen Aldehydgruppen über eine Azomethin-Bindung verbunden werden können. Vorzugsweise sind die erfindungsgemäß verwendeten basenlabilen antigenen Polypeptide bis zu einem pH von etwa 11 stabil, bevorzugt bis zu einem pH von etwa 10, noch bevorzugter bis zu einem pH von etwa 9, am bevorzugtesten bis zu einem pH von etwa 8. Wenn im Zusammenhang mit der vorliegenden Erfindung Polypeptide erwähnt werden, so werden darunter Proteine mit mindestens 6 Aminosäuren in der Kette verstanden. Gleichartig werden unter Polysacchariden Mehrfachzucker mit mindestens 3 Monomereinheiten in der Kette verstanden. Bevorzugt verwendete Polysaccharide sind dabei Mannan, beispielsweise mit einem Molekulargewicht von mindestens 70 kDa und Dextran, beispielsweise mit einem Molekulargewicht von mindestens 70 kDa, besonders bevorzugt mit einem Molekulargewicht von etwa 2000 kDa.

Durch eine Steuerung der Oxidationsrate, z.B. mittels stöchiometrischem Unterschuss an Oxidationsmittel, kann die Menge an für eine Azomethin-Bindung zwischen Träger und Polypeptid zur Verfügung stehenden Aldehydgruppen leicht eingestellt werden.

Vorzugsweise ist dabei das basenlabile antigene Polypeptid ein Vakzinantigen, besonders bevorzugt ein Antikörper, z.B. ein monoklonaler Antikörper, wie der murine monoklonale Antikörper HE2. Eine neuartige Methode der Krebsvakzinierung ist in der Anmeldung PCT/EP00/00174 (Priorität 13.1.1999) "Verwendung von Antikörpern zur Vakzinierung gegen Krebs", beschrieben, deren Offenbarung hier durch Bezugnahme aufgenommen wird. Der darin beschriebene monoklonale Antikörper HE2, der als Vakzin-Antigen in einer Krebsimpfung verwendet wird, dient als Beispiel, jedoch nicht auf dieses eingeschränkt, für die Formulierung eines Impfstoffes nach der hier beschriebenen Methode der Konjugierung an ein partiell oxidiertes hochmolekulares Polysaccharid.

Gemäß einer weiteren bevorzugten Ausführungsform der vorliegenden Erfindung weist das basenlabile antigene Polypeptid dieselbe Bindungsfeinspezifität wie der Antikörper HE2 auf.

Günstig ist auch, wenn zusätzlich zu dem jeweiligen basenlabilen antigenen Polypeptid noch Substanzen konjugiert werden, die eine Verstärkung der Immunantwort bewirken, beispielsweise GM-CSF, IL-2, IL-12 oder Gamma Interferon oder eine Mischung dieser Substanzen.

Weiters wird bevorzugt, wenn das erfindungsgemäße Polysaccharid-Polypeptid Konjugat noch zusätzlich an Aluminiumhydroxid adsorbiert und/oder mit pharmazeutisch annehmbaren Trägern gemischt wird.

Schlussendlich wird bevorzugt, wenn das erfindungsgemäß erhaltene Polysaccharid-Polypeptid Konjugat als Vakzinformulierung zur Verabreichung durch subcutane, intradermale oder intramuskuläre Injektionen formuliert ist, z.B. durch Lösen bzw. Suspendieren des gegebenenfalls auf beispielsweise Aluminiumhydroxid adsorbierten Konjugats in einem geeigneten physiologischen Puffer und dergl.

Allgemein seien folgende Vorteile und spezifische Eigenschaften des erfindungsgemäßen Konjugats angeführt:
- Die über primäre Amine an die aufoxidierten Polysaccharide angekoppelten Bestandteile (Konjugat und Hilf- bzw. Zusatzstoffe) werden in Gegenwart eines Überschusses von Molekülen mit freien primären Aminen, wie beispielsweise Serumproteinen, langsam abgelöst. Der dadurch entstehende "Slow Release" Effekt ist für Impfstoffe gewünscht, da dadurch Antigen-präsentierende Zellen die Impfantigene länger lokal an der Impfstelle aufnehmen können.
- Durch die Wahl des Polysaccharides können die Eigenschaften des Konjugates beeinflusst werden. Das betrifft sowohl die molekulare Größe des Polysaccharides als auch dessen chemische Zusammensetzung. Wird zum Beispiel Mannan als Polysaccharid gewählt, wird das entsprechende Konjugat bevorzugt von Zellen des Immunsystems aufgenommen, die den Mannoserezeptor tragen. Dazu gehören insbesondere Makrophagen und dendritische Zellen als professionelle Antigen-präsentierende Zellen. Dadurch wird eine gesteigerte Immunantwort erreicht.
- Es können an partiell aufoxidierte Polysaccharide gleichzeitig mehrere Komponenten gebunden werden. Das können mehrere unterschiedliche Vakzinantigene sein, oder auch Vakzinantigene zusammen mit Komponenten, die die Immunantwort verstärken, wie zum Beispiel die Proteine GM-CSF, IL-2, IL-12 oder Gamma Interferon.

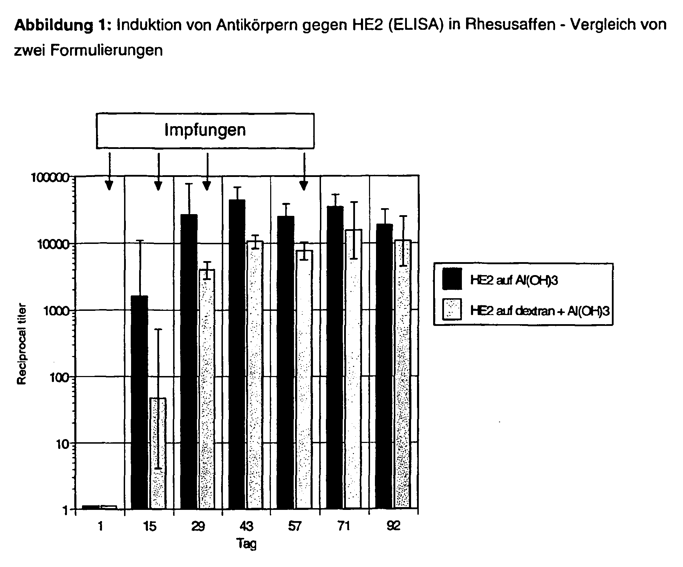

Die beiliegende Abb. 1 zeigt den Vergleich von zwei Formulierungen hinsichtlich der Induktion von Antikörpern gegen HE2 (ELISA) in Rhesusaffen.

### Beispiel :

Zunächst wird Dextran mit einem Molekulargewicht von 2000 kDa (SIGMA D-5376) mit Natriummetaperjodat zu 20% aufoxidiert. Zu diesem Zweck werden 324 mg Dextran in 4 ml destilliertem Wasser unter Rühren gelöst. Zu dieser Lösung werden 86 mg von vorher in 0,6 ml destilliertem Wasser gelösten Natriummetaperjodat zugegeben und 30 min bei 37°C im Dunkeln inkubiert. 25 mg des Antikörpers HE2 (PCT/EP00/00174) werden mit 1 M Na₂HPO₄ auf pH 7,4 gebracht und 45 µl einer Thimerosallösung (10 mg/ml) zugegeben.

Zu dieser Lösung werden 1,675 ml der oben erhaltenen oxidierten Dextranlösung gegeben und 2 Tage bei 37°C im Dunkeln inkubiert. Die Vollständigkeit der Reaktion wird durch Chromatographie an einer Molekulargewichtssäule (Zorbax 450) analytisch überprüft. Das Signal entsprechend einem Molekulargewicht von 150 kDa (monomerer HE2) ist verschwunden und es tritt statt dessen ein Signal im Ausschlussvolumen der Säule auf, das einem Molekulargewicht von > 2000 kDa entspricht.

Die erhaltene Lösung wird mit Hilfe einer präparativen Molekulargewichtssäule chromatographiert, die mit dem endgültigen Puffer (1mM Phosphatpuffer in physiologischer Kochsalzlösung, pH = 5,5) äquilibiert ist. Das im Ausschlussvolumen erhaltene Material besteht aus dem hochmolekularen Konjugat des Antikörpers HE2 an partiell aufoxidiertem Dextran. Der Gehalt an konjugiertem HE2 kann durch Integration des Signales nach analytischer Chromatographie an einer Molekulargewichtssäule im Vergleich zu monomeren HE2 bestimmt werden. Die erhaltene Lösung wird mit einem wässrigen Aluminiumhydroxid so vermischt, dass die Endkonzentration 0,5 mg HE2 an 1,67 mg Aluminiumhydroxid in 0,5 ml Puffer beträgt.

Vier Rhesusaffen wurden an den Tagen 1, 15, 29 und 57 subcutan mit 0.5 ml obiger Formulierung immunisiert. Die Sera von verschiedenen Zeitpunkten wurden mittels ELISA auf Induktion von Antikörpern gegen monomeres HE2 getestet. Als Vergleich wurden vier Rhesusaffen in gleicher Weise mit einer Standardformulierung von 0,5 mg monomeren HE2 adsorbiert an 1,67 mg Aluminiumhydroxid, geimpft.

Der ELISA wurde wie folgt durchgeführt:
100 µl Aliquots des MAK HE2 (Lösung mit 10 µg/ml in Bindungspuffer) werden in den Löchern einer Mikrotiterplatte 1 h bei 37°C inkubiert. Nach sechsmaligem Waschen der Platte mit Waschpuffer A werden je 200 µl des Blockierungspuffers A zugesetzt und 30 min bei 37°C inkubiert. Nach Waschen der Platte wie oben beschrieben werden je 100 µl Aliquots der zu testenden Affensera in Verdünnungen von 1:100 bis 1:1 000 000 in Verdünnungspuffer A 1 h bei 37°C inkubiert. Nach Waschen der Platte wie oben beschrieben werden je 100 µl des Peroxidase-konjugierten Ziegen anti-Human-Ig Antikörpers (Zymed) in einer Verdünnung von 1:1000 in Verdünnungspuffer A zugesetzt und 30 min bei 37°C inkubiert. Die Platte wird viermal mit Waschpuffer A und zweimal mit Färbepuffer gewaschen. Die Antikörperbindung wird durch Zusatz von je 100 µl des spezifischen Substrats nachgewiesen und die Farbreaktion durch Zugabe von je 50 µl Stopplösung nach ca. 10 min abgebrochen. Die Auswertung erfolgt durch Messen der optischen Dichte (OD) bei 490 nm (Wellenlänge der Referenzmessung ist 620 nm).

Die Ergebnisse des ELISA sind in Abbildung 1 dargestellt. Die mit dem Konjugat von HE2 an Dextran geimpften Tiere entwickelten vergleichbare Titer von Antikörpern gegen HE2 wie die mit der Standardformulierung geimpften Affen.

Weiters wurde untersucht, wieweit HE2 nach Applikation im Serum der Affen wiederzufinden ist. Dazu wurde wieder ein ELISA eingesetzt, der wie folgt durchgeführt wurde:
100 µl Aliquots eines gereinigten polyklonalen anti-idiötypischen Ziegenantikörpers gegen HE2 (Lösung mit 10 µg/ml in Bindungspuffer) werden in den Löchern einer Mikrotiterplatte 1 h bei 37°C inkubiert. Nach sechsmaligem Waschen der Platte mit Waschpuffer A werden je 200 µl des Blockierungspuffers zugesetzt und 30 min bei 37°C inkubiert. Nach Waschen der Platte wie oben beschrieben werden je 100 µl Aliquots der zu testenden Affensera in Verdünnungen von 1:4 bis 1:100 000 in Verdünnungspuffer A 1 h bei 37°C inkubiert. Nach Waschen der Platte wie oben beschrieben werden je 100 µl eines Peroxidase-konjugierten Ziegen anti-Maus-IgG Antikörpers (Zymed) in einer Verdünnung von 1:1000 in Verdünnungspuffer zugesetzt und 30 min bei 37°C inkubiert. Die Platte wird viermal mit Waschpuffer und zweimal mit Färbepuffer gewaschen. Die Antikörperbindung wird durch Zusatz von je 100 µl des spezifischen Substrats nachgewiesen und die Farbreaktion durch Zugabe von je 50 µl Stopplösung nach ca. 10 min abgebrochen. Die Auswertung erfolgt durch Messen der optischen Dichte (OD) bei 490 nm (Wellenlänge der Referenzmessung ist 620 nm).

Die Ergebnisse sind in der folgenden Tabelle dargestellt:

| Zeitpunkt | Konjugat Vakzine | Standardformulierung |
|---|---|---|
| 0 | 0; 0; 0; 0 ng/ml | 0; 0; 0; 0 ng/ml |
| 1 h | 0; 0; 0; 0 ng/ml | 13; 17; 74; 280 ng/ml |
| 4 h | 0; 0; 0; 0 ng/ml | 200, 280, 400, 740 ng/ml |
| 24 h | 0; 0; 0; 0 ng/ml | 960, 960, 1000, 740 ng/ml |

Nach 24 h ist bei den Tieren, die das Konjugat geimpft bekommen hatten, eine Andeutung einer HE2-Konzentration im Serum zu erkennen, die aber unter der Nachweisgrenze von ca. 10 ng HE2 / ml Serum liegt. Offensichtlich ist die Desorption des Vakzinantigens durch die Konjugation an partiell oxidiertes Dextran im Vergleich zur Standardformulierung deutlich verringert worden. Dadurch können vermutlich weniger Impfungen zur Erreichung eines vergleichbaren Titers ausreichen als bei einer Standardformulierung auf Aluminiumhydroxid.

### Verwendete Materialien:

| | |
|---|---|
| Mikrotiterplatten: | Immuno Plate F96 MaxiSorp (Nunc) für ELISA |
| Bindungspuffer: | 15 mM Na₂CO₃ |
| | 35 mM NaHCO₃ |
| | 3 mM NaN₃ |
| | pH-Wert: 9,6 |
| | |
| PBS deficient: | 138 mM NaCl |
| | 1,5 mM KH₂PO₄ |
| | 2,7 mM KCI |
| | 6,5 mM Na₂HPO₄ |
| | pH-Wert: 7,2 |
| | |
| Waschpuffer : | 0,05 % Tween 20 in PBS deficient |
| | |
| Blockierungspuffer : | 5 % foetales Kälberserum (hitzeinaktiviert) |
| | in PBS deficient |
| | |
| Verdünnungspuffer: | 2% foetales Kälberserum (hitzeinaktiviert) |
| | in PBS deficient |
| | |
| Färbepuffer: | 24,3 mM Zitronensäure |
| | 51,4 mM Na₂HPO₄ |
| | pH-Wert: 5,0 |
| | |
| Substrat: | 40 mg o-Phenylendiamin Dihydrochlorid |
| | 100 ml Färbepuffer |
| | 20 µl H₂O₂ (30%) |
| | |
| Stopplösung: | 4 N H₂SO₄ |

## Patentansprüche

1. Verfahren zur Herstellung eines Polysaccharid-Polypeptid Konjugates durch Umsetzung eines Polysaccharids mit einem mindestens eine freie Aminogruppe enthaltenden Polypeptid, **dadurch gekennzeichnet, dass** ein vicinale Hydroxylgruppen aufweisender Polysaccharid-Träger unter Ringöffnung zur Schaffung von vicinalen Aldehydgruppen teilweise, vorzugsweise zumindest zu 20%, bei stöchiometrischem Unterschuss eines Oxidationsmittels aufoxidiert und mit einem oder mehreren basenlabilen antigenen Polypeptid(en) enthaltend mindestens eine freie Aminogruppe umgesetzt wird, wobei das bzw. die Polypeptide über mindestens eine Azomethin-Bindung direkt an den Polysaccharid-Träger gebunden werden.

2. Verfahren nach Anspruch 1, **dadurch gekennzeichnet, dass** das bzw. die antigene(n) Polypeptid(e) bis zu einem pH von etwa 11 stabil ist bzw. sind.

3. Verfahren nach Anspruch 1, **dadurch gekennzeichnet, dass** das bzw. die antigene(n) Polypeptid(e) bis zu einem pH von etwa 10 stabil ist bzw. sind.

4. Verfahren nach Anspruch 1, **dadurch gekennzeichnet, dass** das bzw. die antigene(n) Polypeptid(e) bis zu einem pH von etwa 9 stabil ist bzw. sind.

5. Verfahren nach Anspruch 1, **dadurch gekennzeichnet, dass** das bzw. die antigene(n) Polypeptid(e) bis zu einem pH von etwa 8 stabil ist bzw. sind.

6. Verfahren nach einem der Ansprüche 1 bis 5, **dadurch gekennzeichnet, dass** als Polysaccharid Mannan, vorzugsweise mit einem Molekulargewicht von mindestens 70 kDa, verwendet wird.

7. Verfahren nach einem der Ansprüche 1 bis 5, **dadurch gekennzeichnet, dass** als Polysaccharid Dextran, vorzugsweise mit einem Molekulargewicht von mindestens 70 kDa, besonders bevorzugt mit einem Molekulargewicht von etwa 2000 kDa, verwendet wird.

8. Verfahren nach einem der Ansprüche 1 bis 7, **dadurch gekennzeichnet, dass** als basenlabiles antigenes Polypeptid ein Vakzinantigen verwendet wird.

9. Verfahren nach Anspruch 8, **dadurch gekennzeichnet, dass** als Vakzinantigen ein Antikörper verwendet wird.

10. Verfahren nach Anspruch 9, **dadurch gekennzeichnet, dass** als Antikörper ein monoklonaler Antikörper verwendet wird.

11. Verfahren nach Anspruch 10, **dadurch gekennzeichnet, dass** als monoklonaler Antikörper der Antikörper HE2 verwendet wird.

12. Verfahren nach einem der Ansprüche 9 bis 11, **dadurch gekennzeichnet, dass** der Antikörper dieselbe Bindungsfeinspezifität wie der Antikörper HE2 aufweist.

13. Verfahren nach einem der Ansprüche 1 bis 12, **dadurch gekennzeichnet, dass** zusätzlich zu den jeweiligen basenlabilen antigenen Polypeptid noch Substanzen konjugiert werden, die eine Verstärkung der Immunantwort bewirken.

14. Verfahren nach Anspruch 13, **dadurch gekennzeichnet, dass** es sich bei diesen Substanzen um GM-CSF, IL-2, IL-12 oder Gamma Interferon oder um eine Mischung dieser Substanzen handelt.

15. Verfahren nach einem der Ansprüche 1 bis 14, **dadurch gekennzeichnet, dass** das Konjugat noch zusätzlich an Aluminiumhydroxid adsorbiert ist.

16. Verfahren nach einem der Ansprüche 1 bis 15, **dadurch gekennzeichnet, dass** das Konjugat mit einem pharmazeutisch annehmbaren Träger gemischt wird.

17. Konjugat erhältlich nach einem der Ansprüche 1 bis 16.

18. Konjugat nach Anspruch 17, **dadurch gekennzeichnet, dass** es zur Verabreichung durch subcutane, intradermale oder intramuskuläre Injektionen formuliert ist.

## Claims

1. A method of producing a polysaccharide-polypeptide conjugate by reacting a polysaccharide with a polypeptide which comprises at least one free amino group, **characterized in that** a polysaccharide carrier comprising vicinal hydroxyl groups is partially oxidized, preferably by at least 20%, under ring opening at stoichiometric deficiency of an oxidant to create vicinal aldehyde groups and reacted with one or more base-instable antigenic polypeptide(s) containing at least one free amino group, the polypeptide(s) being bound directly to the polysaccharide carrier via at least one azomethine bond.

2. A method according to claim 1, **characterized in that** the antigenic polypeptide(s) is (are) stable up to a pH of approximately 11.

3. A method according to claim 1, **characterized in that** the antigenic polypeptide(s) is (are) stable up to a pH of approximately 10.

4. A method according to claim 1, **characterized in that** the antigenic polypeptide(s) is (are) stable up to a pH of approximately 9.

5. A method according to claim 1, **characterized in that** the antigenic polypeptide(s) is (are) stable up to a pH of approximately 8.

6. A method according to any one of claims 1 to 5, **characterized in that** mannan, preferably having a molecular weight of at least 70 kDa, is used as the polysaccharide.

7. A method according to any one of claims 1 to 5, **characterized in that** dextrane, preferably having a molecular weight of at least 70 kDa, particularly preferred having a molecular weight of approximately 2000 kDa, is used as the polysaccharide.

8. A method according to any one of claims 1 to 7, **characterized in that** a vaccine antigen is used as the base-instable antigenic polypeptide.

9. A method according to claim 8, **characterized in that** an antibody is used as the vaccine antigen.

10. A method according to claim 9, **characterized in that** a monoclonal antibody is used as the antibody.

11. A method according to claim 10, **characterized in that** the antibody HE2 is used as the monoclonal antibody.

12. A method according to any one of claims 9 to 11, **characterized in that** the antibody has the same binding fine specificity as the antibody HE2.

13. A method according to any one of claims 1 to 12, **characterized in that** in addition to the respective base-instable antigenic polypeptides, further substances are conjugated which cause an enhancement of the immune response.

14. A method according to claim 13, **characterized in that** these substances are GM-CSF, IL-2, IL-12 or gamma-interferon or a mixture of these substances.

15. A method according to any one of claims 1 to 14, **characterized in that** the conjugate additionally is adsorbed on aluminum hydroxide.

16. A method according to any one of claims 1 to 15, **characterized in that** the conjugate is mixed with a pharmaceutically acceptable carrier.

17. A conjugate obtainable according to any one of claims 1 to 16.

18. A conjugate according to claim 17, **characterized in that** it is formulated to be administered by subcutaneous, intradermal or intramuscular injections.

## Revendications

1. Procédé de production d'un conjugué polysaccharide-polypeptide par réaction d'un polysaccharide avec un polypeptide contenant au moins un groupe amino libre, **caractérisé en ce qu'**un polysaccharide-support présentant des groupes hydroxyle vicinaux est soumis, avec ouverture du noyau en vue de créer des groupes aldéhyde vicinaux, à une oxydation partielle, avantageusement à 20 % au moins, pour une quantité stoechiométrique déficitaire d'un agent oxydant, et est amené à réagir avec un ou plusieurs polypeptides antigéniques basolabiles contenant au moins un groupe amino libre, le ou les polypeptide(s) se liant alors directement au polysaccharide-support par au moins une liaison azométhine.

2. Procédé suivant la revendication 1, **caractérisé en ce que** le ou les polypeptides antigéniques est ou sont stables jusqu'à un pH égal à 11 environ.

3. Procédé suivant la revendication 1, **caractérisé en ce que** le ou les polypeptides antigéniques est ou sont stables jusqu'à un pH égal à 10 environ.

4. Procédé suivant la revendication 1, **caractérisé en ce que** le ou les polypeptides antigéniques est ou sont stables jusqu'à un pH égal à 9 environ.

5. Procédé suivant la revendication 1, **caractérisé en ce que** le ou les polypeptides antigéniques est ou sont stables jusqu'à un pH égal à 8 environ.

6. Procédé suivant l'une des revendications 1 à 5, **caractérisé en ce que** le polysaccharide utilisé est un mannane, ayant avantageusement un poids moléculaire au moins égal à 70 kDa.

7. Procédé suivant l'une des revendications 1 à 5, **caractérisé en ce que** le polysaccharide utilisé est un dextrane, ayant avantageusement un poids moléculaire au moins égal à 70 kDa, très avantageusement un poids moléculaire d'environ 2000 kDa.

8. Procédé suivant l'une des revendications 1 à 7, **caractérisé en ce que** le polypeptide antigénique basolabile que l'on utilise est un antigène vaccinal.

9. Procédé suivant la revendication 8, **caractérisé en ce qu'**on utilise comme antigène vaccinal un anticorps.

10. Procédé suivant la revendication 9, **caractérisé en ce que** l'anticorps que l'on utilise est un anticorps monoclonal.

11. Procédé suivant la revendication 10, **caractérisé en ce que** l'anticorps monoclonal que l'on utilise est l'anticorps HE2.

12. Procédé suivant l'une des revendications 9 à 11, **caractérisé en ce que** l'anticorps a la même finesse de spécifité de liaison que l'anticorps HE2.

13. Procédé suivant l'une des revendications 1 à 12, **caractérisé en ce que** d'autres substances qui causent une accentuation de la réponse immunitaire sont conjugées en plus du polypeptide antigénique basolabile respectif.

14. Procédé suivant la revendication 13, **caractérisé en ce que** les substances concernées consistent en GM-CSF, IL-2, IL-12 ou Interféron gamma ou en un mélange de ces substances.

15. Procédé suivant l'une des revendications 1 à 14, **caractérisé en ce que** le conjugué est en outre adsorbé sur de l'hydroxyde d'aluminium.

16. Procédé suivant l'une des revendications 1 à 15, **caractérisé en ce que** le conjugué est mélangé à un support pharmaceutiquement acceptable.

17. Conjugué pouvant être obtenu selon l'une des revendications 1 à 16.

18. Conjugué suivant la revendication 17, **caractérisé en ce qu'**il est formulé en vue de son administration par injections par voie sous-cutanée, intradermique ou intramusculaire.
